# EUROPEAN PATENT SPECIFICATION

(11) **EP 1 551 481 B1**
(45) Date of publication and mention of the grant of the patent: **08.04.2009**
(21) Application number: 03756754.2
(22) Date of filing: 16.10.2003
(51) Int. Cl.: A61M 5/32

(54) **INJECTION SYRINGE WITH RETRACTABLE NEEDLE**
INJEKTIONSSPRITZE MIT EINZIEHBARER NADEL
SERINGUE D'INJECTION A AIGUILLE RETRACTABLE

(30) Priority: 18.10.2002 NL 1021689; 18.10.2002 US 419678 P
(43) Date of publication of application: 13.07.2005
(73) Proprietor: ADVANCED PROTECTIVE INJECTION SYSTEMS B.V., 2514 BK Den Haag (NL)
(72) Inventor: GOOSSENS, Bjorn, Olaf, 2514 BK Den Haag (NL); LUCAS, Jan, Hendrik, 2960 Brecht/St. Job (BE)
(74) Representative: Riemens, Roelof Harm
(86) International application number: PCT/NL2003/000699
(87) International publication number: WO 2004/035120

(56) References cited:
- WO-A-00/27450
- WO-A-02/26295
- GB-A- 2 332 628
- US-A- 5 221 262
- US-B1- 6 432 082

## Description

The present invention relates to an injection syringe with retractable needle. Injection syringes of this type are known in practice see e.g. U.S. patent 5,221,262 disclosing the features of the preamble of claim 1. After the liquid which is present in the liquid container of the injection syringe has been ejected, to allow the needle to be retracted, by way of example the piston head of the piston and the needle mount of the needle are coupled to one another. As a result of the piston then being moved back again, the needle can be pulled back into the liquid container of the injection syringe, so that it is possible to prevent the risk of injury on the needle. In addition, this ensures that the injection syringe is not reused.

One drawback of the known injection syringes with retractable needle is that the coupling means used are generally fairly fragile.

Another drawback of the known injection syringes is that the needle, when the needle is being fitted to the outlet opening of the liquid container, is often difficult to position, i.e. in the known injection syringes the coupling means have to be fitted very accurately through the outlet opening of the liquid container. If this is not done carefully, as is often the case in everyday use, this may result in the needle not being stably secured to the outlet opening of the liquid container and/or the coupling means being damaged in some way, and consequently it is impossible to ensure that the needle is safely retracted after the injection syringe has been used.

WO 02/26295, in the name of Nicodel S.A., describes an injection syringe with retractable needle. According to WO 02/26295, the retractable nature of the needle is brought about by coupling of the piston head and needle mount. During this coupling process, the needle mount in the liquid container is unblocked. A drawback of the injection syringe described in WO 02/26295 is that the needle mount has already been unblocked and thereby released while the coupling between needle mount and piston head is taking place or after this coupling has taken place, but before the piston head has been moved back into the liquid container. As a result, the user loses accurate control of the action of the injection syringe, which is a highly undesirable state of affairs. This problem arises in particular if the needle is still in the patient. If the needle can start to move as a result of the needle mount being unblocked, this may be very painful to the patient. It will be clear that this is highly undesirable.

It is an object of the present invention to provide a solution to the above and other drawbacks.

It is a further object of the present invention to provide a leakage-free injection syringe with retractable needle which is easy to produce and also easy to use.

Another object of the present invention is to provide a leakage-free injection syringe which can be produced with the minimum possible number of components and as inexpensively as possible. These objects are achieved by the present invention by an injection syringe with retractable needle as defined in claim 1.

The injection syringe with retractable needle according to the invention is simple and inexpensive to produce on account of its simple design and the small number of components.

A significant advantage of the injection syringe according to the invention is that the blocking means for blocking the needle mount in the outlet opening of the liquid container - unalike in the case, for example, of the injection syringe described in WO 02/26295 - is only unblocked as a result of the needle mount being retracted into the liquid container. This results in very precise control of the operation of the injection syringe.

This retraction of the needle which is intended in accordance with the invention can of course be realized in a wide range of ways.

According to the present invention, it is particularly advantageous if the needle mount can be mounted both "around the outside" (as a result of the needle mount being placed in the outlet opening from the outside of the liquid container) and "through the inside" (as a result of the needle mount being put in place via the inside of the liquid container).

The invention will be explained in more detail below, without any limitation being implied, on the basis of an injection syringe which is provided with a piston in the liquid container.

The blocking means is designed in the form of one or more resilient lugs on the needle mount, which can be received in corresponding recesses in the liquid container.

This prevents the needle, when it has been secured to the outlet opening of the liquid container, from unintentionally moving into the liquid container during use (for example during injection). It has been found that the injection syringe according to the present invention can withstand relatively high forces, on account of the blocking means, without the needle unintentionally moving into the liquid container. Therefore, the needle mount with the needle can only enter the liquid container once the blocking means has been unblocked. The person skilled in the art will understand that the blocking means may be designed in various ways for this purpose, provided only that it is possible to provide for unblocking after the liquid container has been emptied and the needle mount has been introduced into the liquid container.

For this purpose, by way of example, a recess in the needle container and a projecting part on the needle mount, which can interact with one another, can be provided. Alternatively, the needle mount may comprise a recess and the liquid container a projecting part.

Furthermore, according to the invention, the injection syringe also comprises a piston movable inside the said liquid container and has a piston head, to which a piston rod is secured;
in which the needle mount of the needle and the piston head comprise coupling means which are designed so that they can be unmistakably coupled to one another in order that, after the piston head has been moved completely into the liquid container in order for liquid which is present in the liquid container to be discharged via the outlet opening, the needle can be retracted into the liquid container after the piston head has been coupled to the needle mount of the needle and the blocking means has been unblocked;
wherein the blocking means can only be unblocked by the piston being moved away from the outlet opening after the needle mount has been coupled to the piston head.

This results in very simple unblocking of the blocking means.

When the injection syringe according to the invention is being used, after the liquid container has been emptied as a result of the piston being moved towards the outlet opening, a coupling is brought about between the piston head and the needle mount of the needle. Only then is the needle mount of the needle released from the outlet opening of the liquid container, specifically as a result of the piston head being moved away from the outlet opening again with the aid of the piston rod. As a result, the needle mount together with the needle is retracted into the liquid container. The coupling is preferably an "unmistakable" coupling, i.e. is not produced by clamping or friction, for example. This is because coupling which is based on friction may be insufficient for the needle mount with needle to be retracted back into the liquid container after the liquid container has been emptied.

The coupling means of the needle mount can be designed in various ways.

It is particularly expedient if the coupling means of the piston head are designed in the form of a recess with an inwardly directed collar.

Furthermore, the coupling means of the needle mount of the needle comprise at least two ribs which are connected to one another at a connection point on the side which faces the piston head, and in which the coupling means of the needle mount comprise a coupling element which is directed towards the wall of the liquid container for unmistakable coupling to the collar of the piston head.

This makes the needle mount with needle very easy to fit into or onto the outlet opening of the liquid container of the injection syringe, with the risk of damage to the coupling means of the needle mount and the piston head, which may occur with the known injection syringes, being substantially minimized. It has been found that the needle mount with needle can also be positioned in or on the outlet opening automatically, which has hitherto caused problems.

The presence of the connection point prevents one or more of the ribs of the needle mount being damaged during assembly of the injection syringe. The person skilled in the art will understand that it is also possible to use more than two ribs. According to a very advantageous embodiment, the needle mount comprises three ribs which all meet at the connection point. The ribs may be of any suitable form and are generally designed in such a manner that they form a connection between that part of the needle mount in which the needle is secured, on the one hand, and the connection point which is located at a position of the needle mount which faces the piston head, on the other hand.

Furthermore, the person skilled in the art will understand that the connection point may be designed in a wide range of ways, provided only that the intended effect is achieved. Obviously, the connection point may also be a connection zone. It is preferable for the connection point to comprise a hinged connection, in particular what is known as an "integral hinge". Integral hinges of this type are known per se in plastics technology and therefore require no further explanation in the present application.

Furthermore, the coupling means of the needle mount preferably comprise a coupling element which substantially faces the wall of the liquid container and may be designed in a wide range of ways, provided only that unmistakable coupling to the collar of the piston head is achieved. It is also possible for the collar to be designed in a wide range of ways for this purpose. According to a very advantageous embodiment, at least one of the ribs comprises a coupling element of this type.

In general, it is the case that the injection syringe according to the present invention is very easy and safe to use, without any complicated actions having to be carried out.

Yet another advantage of the injection syringe according to the invention is that the coupling of the piston head and the needle mount is less critical in terms of dimensions, resulting in production engineering advantages. It should also be noted that the coupling means of the injection syringe according to the present invention create a particularly reliable and stable coupling of the piston head to the needle mount.

The person skilled in the art will understand that the coupling mechanism according to the present invention is not restricted to a "normal" injection syringe. By way of example, the coupling mechanism according to the present invention is also useful for other types of injection syringes, such as for example prefilled injection syringes, luer-lock syringes, etc., provided only that the needle is retracted into the liquid container after the liquid container has been emptied.

The person skilled in the art will understand that the needle with needle mount can be secured in or on the outlet opening in a wide range of ways, provided only that it is secured safely and stably.

Furthermore, it is preferable if the ribs of the needle mount, when the needle is retracted into the liquid container as a result of the piston head being coupled to the needle mount, can be moved closer together. The result of this is that, as soon as the piston head has been coupled to the needle mount, the blocking means is unblocked simply yet effectively in order to allow the needle to be retracted into the liquid container.

The person skilled in the art will quickly understand that for this purpose the ribs may be designed in various ways, provided only that it is possible for the ribs (in particular in the vicinity of the blocking means) to be moved closer together when the needle is retracted into the liquid container. For this purpose, by way of example, the ribs may comprise a hinge or curvature. It is preferable for the ribs and the connection point to be designed in such a manner that together they are substantially oval-shaped or elliptical. Furthermore, it is preferable for the ribs to be resilient.

The coupling means of the needle mount can be produced particularly easily. For example, that side of the needle mount which faces the piston head may, for example, be designed as a substantially cylindrical body in which there is a continuous opening. The ribs which are formed as a result in this case partially surround the continuous opening and at one end are secured to that part of the needle mount in which the needle is secured, while at the other end they meet at the connection point.

According to a further embodiment according to the present invention, at least one of the ribs of the needle mount has a curvature in the direction of the longitudinal axis of the liquid container. The result of this is that when the needle is retracted into the liquid container, the needle is positioned at an angle with respect to the longitudinal axis of the liquid container. If the piston head is then moved in the direction of the outlet opening of the liquid container, the needle will remain in the liquid container. It is preferable for the curvature of the at least one rib in this case to have a radius of curvature which is such that the needle, after the needle has been retracted into the liquid container, is inclined in the direction of a reinforced part of the liquid container, which substantially prevents the liquid container from being penetrated by the needle. The person skilled in the field will know how to select a suitable radius of curvature depending on the dimensions and design of the injection syringe.

As has also been stated above, according to the invention it is preferable if at least one of the ribs of the needle mount comprises the coupling element which is directed towards the wall of the liquid container.

According to an alternative embodiment, at least two of the ribs of the needle mount comprise a coupling element which is directed towards the wall of the liquid container, at least one of the coupling elements being at a different distance from the connection point compared to the at least one other coupling element. This also makes it surprisingly easy for the needle, after it has been retracted into the liquid container, to be inclined towards a reinforced part of the liquid container, with the result that penetration of the liquid container by the needle is substantially prevented.

Preferably, the coupling means of the needle mount comprise three ribs which are connected to one another at a connection point on the side which faces the piston head.

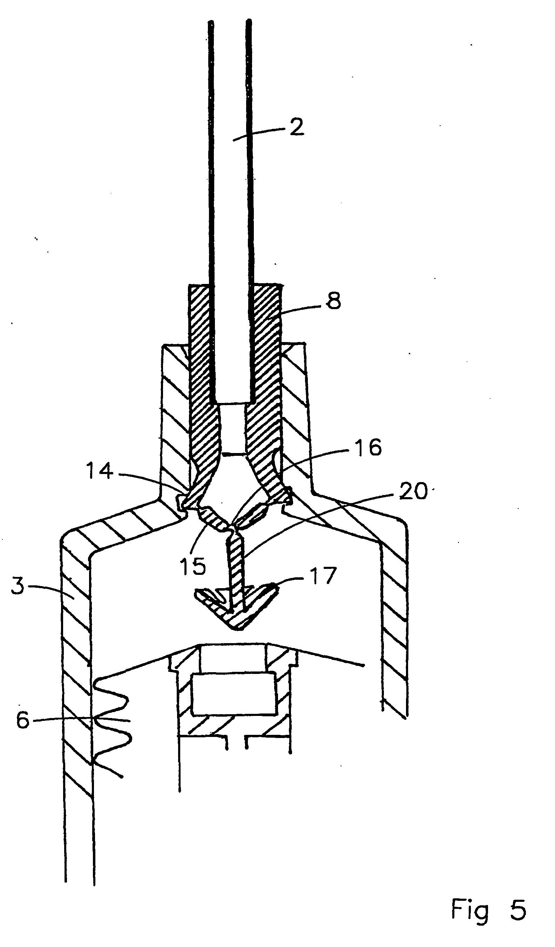

Advantageously, the needle mount is provided, on its side which faces the piston head, with a coupling member for coupling to the piston head, which coupling member is preferably connected to the connection point. This coupling member, which in turn may be designed in various ways, ensures that a good coupling is obtained between needle mount and piston head. In this context, it is particularly advantageous if the coupling member can interact with ribs of the needle mount in such a manner that, as solon as the coupling member of the needle mount has been coupled to the piston head of the piston, the ribs of the needle mount are brought closer together as the piston moves away from the outlet opening, with the result that reliable unblocking of the blocking means is provided (cf. also Figure 5 below).

Furthermore, according to the present invention it is preferable if the injection syringe also comprises a spring member which can force the needle mount with needle into the liquid container after the blocking means has been unblocked.

This also ensures that, after the blocking means has been unblocked, the needle mount with needle can enter the liquid container.

A particular advantage of the use of the spring member is that if appropriate it is possible to dispense with a coupling between the piston head and needle mount in order to enable the needle mount with needle to be moved into the liquid container.

The spring member is preferably positioned in such a way that contact between the spring member and the liquid which is present in the liquid container is prevented.

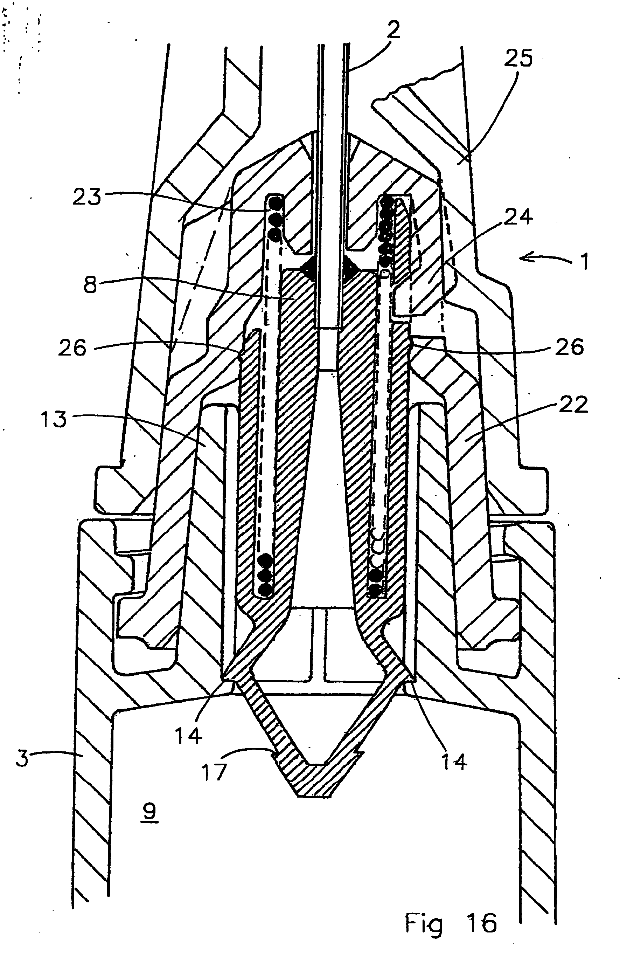

Furthermore, it is advantageous if the spring member can be blocked, preferably by a spring member-blocking means. This prevents unintentional pressure being applied to the needle mount by the spring member. In this case, the spring member-blocking means can also function as a "prestressing means"; if the spring member is to be fitted in the injection syringe by the user himself, the spring member remains in the desired position during assembly provided that the spring member-blocking means has not yet been deactivated, for example by removal of a protective cap (cf. Figure 16).

According to a particularly simple and expedient embodiment, the spring member-blocking means can interact with a protective cap for the purpose of blocking the spring member. Also, the spring member-blocking means preferably forms part of a securing element which can secure the needle mount to a liquid container from the outside in a liquid tight manner.

The present invention also relates to a needle mount and a securing element which are clearly intended for the injection syringe according to the invention.

The present invention will be explained in more detail below with reference to the appended, non-limiting drawing, in which:
- Figure 1 shows a diagrammatic cross section through an injection syringe according to the invention;
- Figure 2 shows a diagrammatic cross section through the injection syringe shown in Fig. 1, in which the needle mount has been coupled to the piston head, but the blocking means has not yet been unblocked;
- Figure 3 shows a diagrammatic cross section through the injection syringe shown in Fig. 1 and Fig. 2, with the needle having been retracted completely into the liquid container;
- Figure 4 shows a perspective view of a needle mount which is suitable for the injection syringe 1 shown in Figs 1-3;
- Figure 5 shows a diagrammatic cross section through an alternative embodiment of the injection syringe according to the invention;
- Figure 6 shows a diagrammatic cross section through a further alternative embodiment of the injection syringe according to the invention;
- Figure 7 shows a perspective view of the needle mount shown in Figure 6;
- Figure 8 shows a perspective view of (a part of) the piston shown in Figure 6;
- Figure 9 shows a diagrammatic cross section through a further alternative embodiment of the injection syringe according to the invention;
- Figure 10 shows a perspective view of (a part of) the piston shown in Figure 9;
- Figure 11 shows a diagrammatic cross section through the injection syringe shown in Figure 9, in which the needle mount has been coupled to the piston head but the blocking means has not yet been unblocked;
- Figure 12 shows a perspective view of the needle mount shown in Figures 9 and 11;
- Figure 13 shows a diagrammatic cross section through a further alternative embodiment of the injection syringe according to the invention;
- Figure 14 shows a perspective view of the needle mount shown in Figure 13;
- Figure 15 shows a diagrammatic cross section through an alternative embodiment of an injection syringe according to the present invention which is provided with a spring member; and
- Figure 16 shows a diagrammatic cross-sectional view of an injection syringe which is similar to that shown in Fig. 15 and also comprises a spring member-blocking means.

### Identical reference numerals denote similar components.

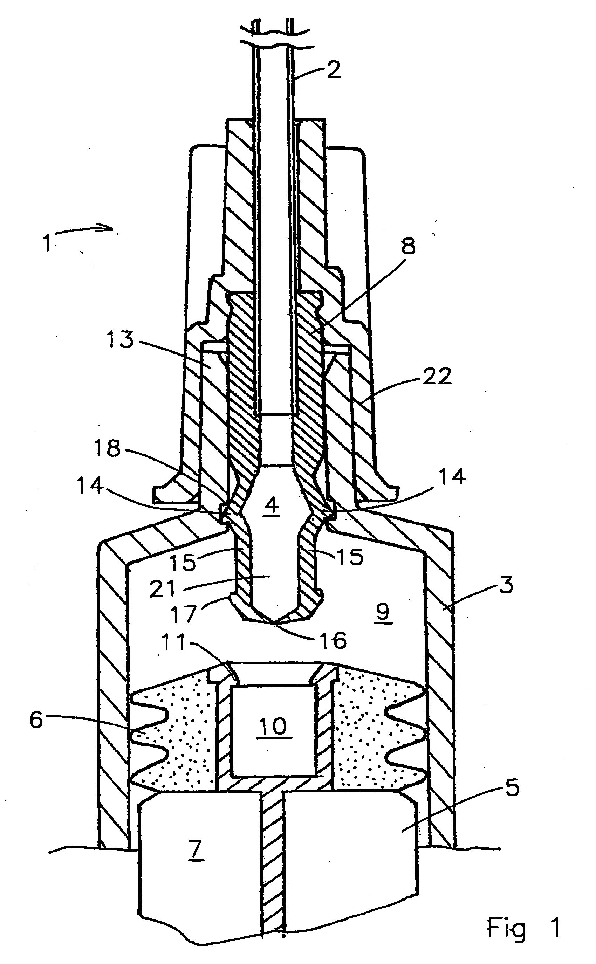

Figure 1 shows a diagrammatic cross section through an injection syringe 1 with retractable needle 2 according to the present invention. The injection syringe 1 comprises a liquid container 3 with an outlet opening 4. In the liquid container 3 there is a displaceable piston 5 with a piston head 6, to which a piston rod 7 is secured. The needle 2 with needle mount 8 is secured in the outlet opening 4 of the liquid container 3, in the embodiment shown with the aid of a securing element 22. The securing element 22, which is known per se, may be designed in various ways and may be provided, for example, with a Luer-lock mechanism.

The needle mount 8 of the needle 2 and the piston head 6 comprise coupling means which are designed so that they can be unmistakably coupled to one another in order that, after the piston head 6 has been moved fully into the liquid container 3 in order to discharge liquid 9 which is present in the liquid container 3 via the outlet opening 4, the needle 2, after the piston head 6 has been coupled to the needle mount 8 of the needle 2, can be retracted into the liquid container 3. This means that after the injection syringe 1 has been used, the user can no longer easily be injured on the needle 2. This also prevents the injection syringe 1 from being reused. The term "unmistakably" coupled to one another means that a reliable coupling is created, i.e. not a coupling which is based on friction or clamping.

The outlet opening 4 of the liquid container 3 comprises a narrowing 13. The narrowing 13 forms part of the liquid container 3 and is fixedly connected thereto, so that it contributes to improving the seal of the injection syringe 1.

The outlet opening 4 of the liquid container 3 comprises a recess 18 for receiving a blocking means 14 in the transition from the liquid container 3 to the narrowing 13. This blocking means 14 is designed to block the needle mount 8 in the outlet opening 4 during injection and can be unblocked by the piston head 6 being coupled to the needle mount 8 of the injection syringe 1 and the piston 5 being retracted. The presence of the blocking means 14 means that a relatively great force can be exerted on the needle 2 when liquid is being injected into a patient without there being any risk of the needle mount 8 being pushed out of the outlet opening 4 into the liquid container 3.

For this purpose, in Fig. 1 the blocking means 14 is designed in the form of a resilient lug which is resilient in the direction of the longitudinal axis of the liquid container 3 when the piston head 6 is pressed onto the outlet opening 4.

In the embodiment shown, the coupling means of the piston head 6 are designed in the form of a recess 10 with an inwardly directed collar 11.

The coupling means of the needle mount 8 comprise two ribs 15 which are connected to one another at a connection point 16 on the side which faces the piston head 6. The ribs (or bridge elements) 15 partially surround a continuous opening 21. At one end, the ribs 15 are secured to that part of the needle mount 8 in which the needle 2 is secured, while at the other end they meet at the connection point 16.

In the embodiment shown in Fig. 1, both ribs 15 comprise a coupling element 17, which substantially faces the wall of the liquid container 3, for unmistakable coupling to the collar 11 of the piston head 6.

The needle mount 8 with the needle 2 is easy to mount on the narrowing 13, with the risk of damage to the ribs 15 of the needle mount 8 being minimized. A securing element 22 (as also shown in Fig. 1) is preferably used to fixedly secure the needle mount 8 to the narrowing 13.

The person skilled in the art will quickly understand that it is also possible to use three or more ribs instead of two ribs 15. Not all the ribs necessarily have to include a coupling element 17, although according to the present invention it is preferable if they do.

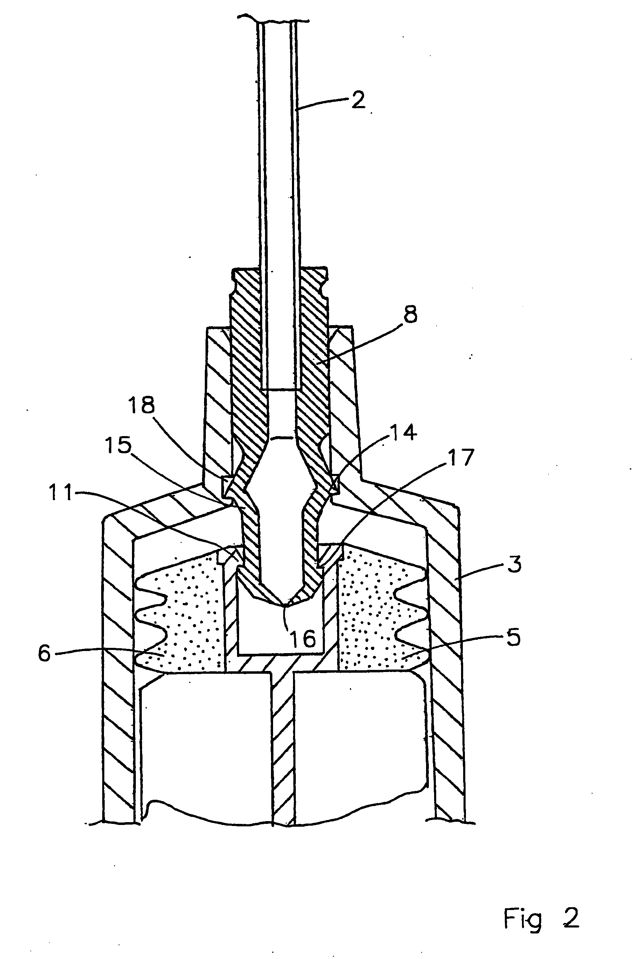

Figure 2 shows a (simplified - the securing element 22 has been omitted for the sake of clarity) diagrammatic cross section through the injection syringe 1 shown in Fig. 1, in which the needle mount 8 has been coupled to the piston head 6 but the blocking means 14 has not yet been unblocked. The person skilled in the art will understand that the injection syringe 1 according to the invention can be of any desired design, providing only that the blocking means 14 is unblocked as a result of the needle mount 8 being coupled to the piston head 6 and then the needle mount 8 being retracted into the liquid container 3.

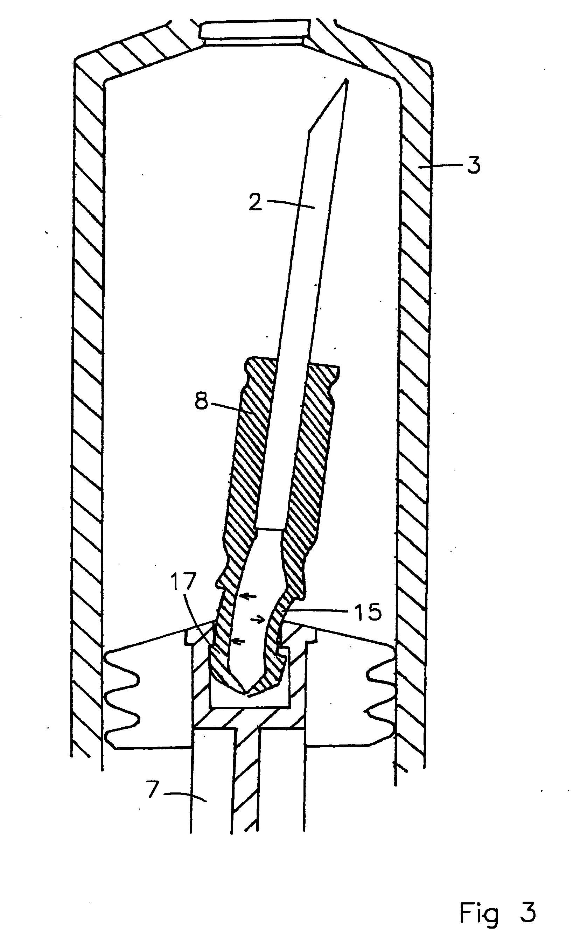

Figure 3 shows a diagrammatic cross section through the injection syringe 1 shown in Fig. 1 and Fig. 2, in which the needle 2 has been retracted into the liquid container 3.

When the injection syringe 1 shown in Figs 1-3 is in use, the injection syringe 1 will be emptied as a result of the liquid 9 which is present in the liquid container 3 being forced through the needle 2 by the piston 5 being moved towards the outlet opening 4. When the injection syringe 1 is completely empty, i.e. when the piston head 6 has been pressed onto the outlet opening 4, the piston head 6 of the piston 5 will be coupled to the needle mount 8, as shown in Fig. 2. In the process, the piston head 6 and the needle mount 8 will interact and provide unmistakable coupling. The blocking means 14, which is designed to block the needle mount 8 in the outlet opening 4, so that the needle mount 8 with needle cannot unintentionally move into the liquid container 3, is at that time (cf. Fig. 2) not yet unblocked. The blocking means 14 is only unblocked as soon as the piston head 6 is moved back away from the outlet opening 4; as a result, the ribs 15 are moved closer together, with the result that the blocking means 14 is unblocked.

If the piston 5 is then pulled back away from the outlet opening 4 with the aid of the piston rod 7, the needle mount 8 with needle 2 will as a result be pulled completely into the liquid container 3 and will not easily be able to move out of it again, as shown in Fig. 3. This prevents reuse of the needle 2. Moreover, this also minimizes the risk of injury on the needle 2 and also the risk of transmission of infectious diseases.

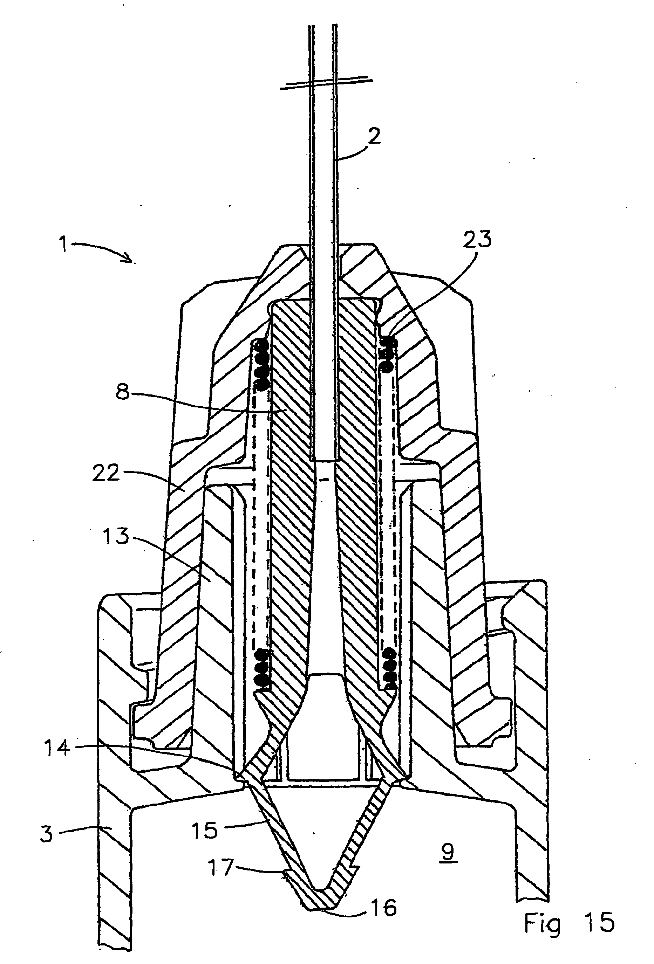

If desired, the injection syringe 1 may be provided with a spring member (not shown; cf. also Figs 15 and 16), so that after the blocking means 14 has been unblocked, the needle mount 8 with needle 2 can be pushed or pulled into the liquid container 3 by this spring member.

One significant advantage of the injection syringe 1 according to the invention is that in use reliable and readily controllable unblocking of the blocking means 14 can be obtained. The blocking means 14 is only unblocked after the needle mount 8 has been coupled to the piston head 6 and after the needle mount 8 and needle 2 have been retracted slightly into the liquid container 3 by means of the piston 5. By pulling on the piston 5 (with the needle mount 8 coupled to it), the ribs 15 - in particular if they are of curved design and, for example, define the shape of an oval - are moved closer together, so that the blocking means 14 is unblocked.

In the embodiment shown in Fig. 3, one of the ribs 15 has a curvature in the direction of the longitudinal axis of the liquid container 3, with the result that the needle 2 comes to stand at an angle with respect to the said longitudinal axis. It is preferable for the curvature of the at least one rib 15 to have a radius of curvature which is such that the needle 1, after it has been retracted into the liquid container 3, is inclined towards a reinforced part (the narrowing 13 in Fig. 3) of the liquid container 3, so that penetration of the liquid container 3 by the needle 2 is substantially prevented.

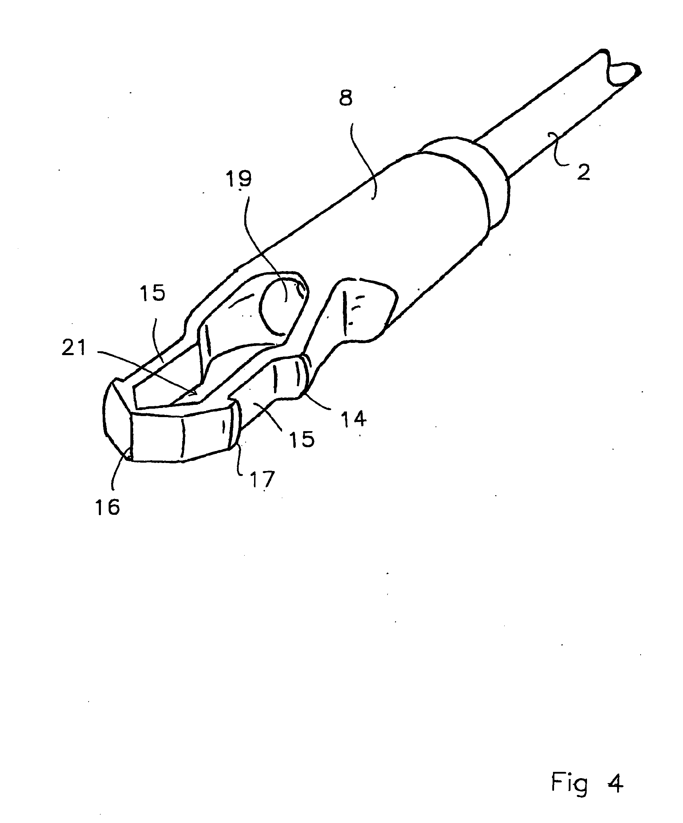

Figure 4 shows a perspective view of a needle mount 8 which is suitable for the injection syringe 1 shown in Figs 1-3. It is clearly apparent that the two ribs 15 are connected at the connection point (or zone) 16. The ribs 15 in this case partially surround the continuous opening 21. At one end, the ribs 15 are secured to that part of the needle mount 8 in which the needle 2 is secured, while at the other end they meet at the connection point 16.

The connection point 16 is in this case designed as what is known as an 'integral hinge'. Furthermore, Fig. 4 shows a passage 19 through which liquid can pass from the liquid container into the needle 2.

Figure 5 shows a diagrammatic cross section through an alternative embodiment of the injection syringe 1 according to the invention. In this case, the coupling means of the needle mount 8 comprise a member 20 which is substantially in the shape of an inverted umbrella and is connected to the connection point (or zone) 16. In the embodiment shown in Fig. 5, the coupling elements 17 are positioned not on the ribs 15, but rather on the member 20.

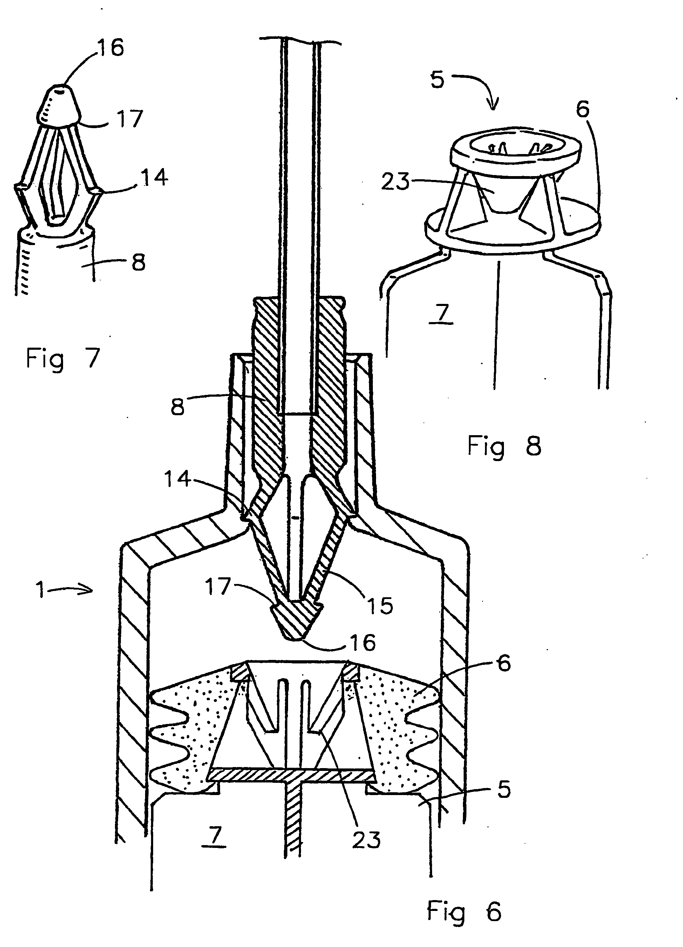

Figure 6 shows a diagrammatic cross section in simplified form (i.e. without securing element 22) through a further alternative embodiment of the injection syringe 1 according to the invention. In this case, the coupling means of the needle mount 8 comprise three ribs 15 which are connected at the connection point (or zone) 16. The coupling elements 17 are positioned in the vicinity of the connection point 16.

Figure 7 shows a perspective view of the needle mount 8 shown in Figure 6, and Figure 8 shows a perspective view of (a part of) the piston 5 shown in Fig. 6.

As can be seen clearly from Fig. 8, the piston head 6 of the piston 5 comprises resilient lips 23 which, during coupling to the needle mount 8, can move towards the side wall of the liquid container 3. As soon as the solid head of the connection point or zone 16 has moved passed the lips 23 during the coupling operation, the lips 23 and the coupling elements 17 of the connection point 16 of the needle mount 8 together provide unmistakable coupling.

A particular advantage of the embodiment shown in Fig. 6-8 is that the underside of the needle mount 8 can be of relatively "rigid" design, so that good blocking of the needle mount 8 in the outlet opening 4 can be obtained. When the needle mount 8 has been coupled to the piston head 6, the ribs 15, as the piston 5 moves away from the outlet opening, will be moved towards one another just enough for it to be possible for the blocking means 14 to be unblocked.

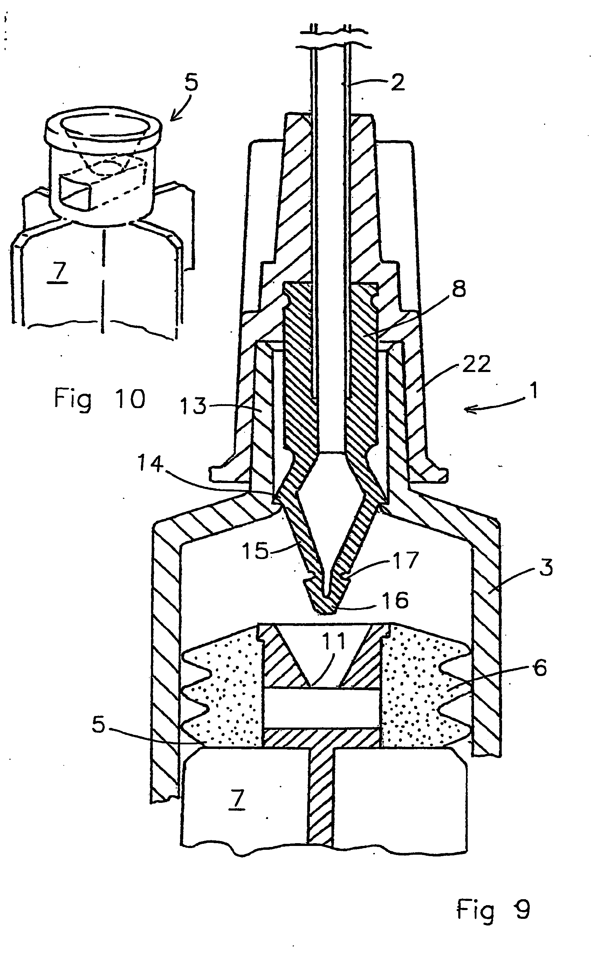

Figure 9 shows a diagrammatic cross section through an alternative embodiment of the injection syringe 1 according to the present invention, in which the piston head 6 is matched to the V-shaped design of the underside of the needle mount 8.

Figure 10 shows a perspective view of (a part of) the piston 5; for the sake of clarity, only that section of the piston head 6 which, in use, interacts with the needle mount 8 in order to obtain coupling is shown.

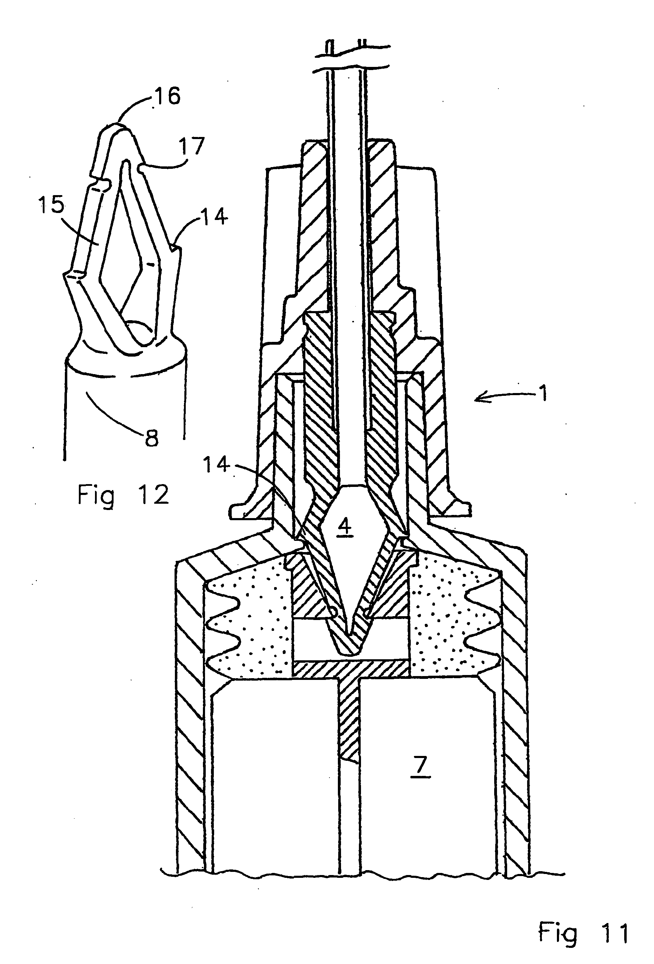

Figure 11 shows the injection syringe 1 shown in Fig. 9 with the needle mount 8 and piston head 6 in the coupled state without the blocking means 14 yet having been unblocked. This will only occur when the piston head 6 is moved away from the outlet opening 4.

For the sake of completeness, Fig. 12 also shows a perspective view of the underside of the needle mount 8 shown in Figs 9 and 11.

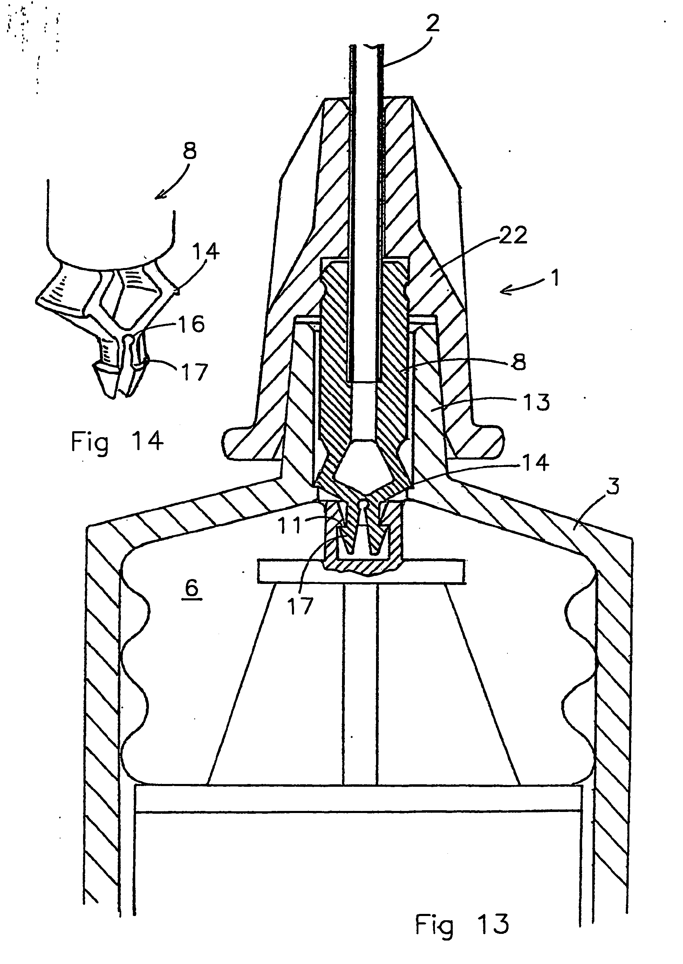

Figure 13 shows a further alternative embodiment of the injection syringe 1 according to the present invention. Figure 14 shows part of the needle mount 8 shown in Fig. 13.

Finally, Fig. 15 and Fig. 16 respectively show a diagrammatic cross section through another alternative embodiment of the injection syringe 1 according to the invention, in which the injection syringe 1 has been provided with a spring member 23 for moving the needle mount 8 into the liquid container 3 after the blocking means 14 has been unblocked.

In the embodiment shown in Fig. 15, the spring member 23 forms part of the needle mount 8.

A particular advantage of the presence of the spring member 23 is that this does not per se require the use of coupling means (for coupling the needle mount 8 to the piston head 6 - cf. for example Fig. 1) in order to enable the needle mount 8 with needle 2 to be moved into the liquid container 3.

Finally, Fig. 16 shows a partial cross-sectional view of an injection syringe 1 according to the invention, in which the injection syringe 1 is provided with a blocking means 24 for the spring member 23. In the embodiment shown, the spring member-blocking means 24 forms part of the securing element 22 and is designed in lip form. In use, the spring member-blocking means 24 is unblocked as soon as the protective cap 25, which is known per se (and is only partially shown in Fig. 16 and protects the needle 2 from damage) is removed. On account of the fact that the blocking means 14 is not yet unblocked, the spring member 23 will not yet be able to push the needle mount 8 back into the liquid container 3; this only occurs after the needle mount 8 has been released from the outlet opening 4 as a result of the blocking means 14 being unblocked (cf. also the explanation given above) and the piston 5 (not shown in Fig. 16) being moved away from the outlet opening 4.

Furthermore, Fig. 16 shows an annular seal 26, which forms part of the needle mount 8. The seal 26 prevents contact between the spring member 23 and liquid 9 which is present in the liquid container 3. Of course, the seal 26 may also form part of the securing element 22, the narrowing 13 or be a separate o-ring which is arranged in a sealing manner so as to prevent contact between the spring member 23 and the liquid 9.

During assembly of the injection syringe 1, the spring member-blocking means 24 can function as a "prestressing means"; if the spring member 23 is introduced into the injection syringe 1 by the user himself (as a result of the needle mount 8 with needle 2, protective cap 25 and securing element 22 being fitted to the liquid container 3), the spring member 23 remains in the desired position during assembly as long as the spring member-blocking means 24 has not yet been deactivated, for example by a protective cap 25 being removed. For the sake of simplicity, a set comprising a protective cap 25, needle mount 8 with needle 2, spring member 23 and securing element 22 can be supplied by the manufacturer in order to facilitate assembly by the user. As soon as the protective cap 25 is removed by the user after the injection syringe 1 has been assembled, the spring member-blocking means 24 is released (cf. dashed line in Fig. 16), with the result that the spring member 23 can exert its action, which is in this case a pushing action on the underside of the needle mount 8. In this case, the needle mount 8 can only be removed from the outlet opening 4 after the blocking means 14 has been unblocked (i.e. after the liquid container 3 has been emptied).

## Claims

1. Injection syringe (1) with retractable needle (2), at least comprising:
- a liquid container (3) with an outlet opening (4);
- a needle (2) with needle mount (8) which is secured on the outlet opening (4) of the liquid container (3);
- a piston (5) movable inside the said liquid container (3) and having a piston head (6), to which a piston rod (7) is secured, wherein the needle mount (8) of the needle (2) and the piston head (6) comprise coupling means which are designed so that they are unmistakably couplable to one another by moving the piston (5) towards the outlet opening (4);
**characterized in that**
a blocking means (14) is provided which is designed to block the needle mount (8) in the outlet opening (4), the blocking means (14) being designed in the form of one or more resilient lugs on the needle mount (8) which are received in corresponding recesses (18) in the liquid container (3),
which blocking means (14) can only be unblocked by retraction of the needle mount (8) and needle (2) into the liquid container (3) by movement of the piston (5) away from the outlet opening (4) after the needle mount (8) has been coupled to the piston head (6),
and wherein the coupling means of the needle mount (8) of the needle (2) comprise at least two ribs (15) which are connected to one another at a connection point (16) on the side which faces the piston head (6), the one or more lugs being provided on the ribs (15),
which ribs (15) of the needle mount (8) are movable closer together, when the needle (2) is retracted into the liquid container (3) by movement of the piston (5) away from the outlet opening (4) after the piston head (6) has been coupled to the needle mount (8).

2. Injection syringe (1) according to claim 1, in which the ribs (15) are resilient.

3. Injection syringe (1) according to one of the preceding claims, in which the ribs (15) partially surround a continuous opening (21).

4. Injection syringe (1) according to one of the preceding claims, in which the connection point (16) comprises a hinged connection, in particular an integrals hinge.

5. Injection syringe (1) according to one or more of the preceding claims, in which at least one of the ribs (15) of the needle mount (8) has a curvature in the direction of the longitudinal axis of the liquid container (3).

6. Injection syringes (1) according to one or more of the preceding claims, in which at least one of the ribs (15) of the needle mount (8) comprises a coupling element (17) which is directed towards the wall of the liquid container (3).

7. Injection syringe (1) according to claim 6, in which at least two of the ribs (15) of the needle mount (8) comprise a coupling element (17) which is directed towards the wall of the liquid container (8), at least one of the coupling elements (17) being at a different distance from the connection point (16) compared to the at least one other coupling element (17).

8. Injection syringe (1) according to one or more of the preceding claims, in which the coupling means of the needle mount (8) comprise three ribs (15) which are connected to one another at the connection point (16) on the side which faces the piston head (6).

9. Injection syringe (1) according to one or more of the preceding claims, in which the needle mount (8) is provided, on its side which faces the piston head (6), with a coupling member (20) for coupling to the piston head (6), which coupling member (20) is preferably connected to the connection point (16).

10. Injection syringe (1) according to one or more of the preceding claims, in which the injection syringe (1) also comprises a spring member (23) for forcing the needle mount (8) with needle (2) into the liquid container (3) after the blocking means (14) has been unblocked.

11. Injection syringe (1) according to claim 10, in which the spring member (23) forms part of the needle mount (8).

12. Injection syringe (1) according to claim 10 or 11, in which the spring member (23) is blocked by a spring member-blocking means (24).

13. Injection syringe (1) according to claim 12, in which the spring member-blocking means (24) interacts with a protective cap (25) for the purpose of blocking the spring member (23).

14. Injection syringe (1) according to claim 12 or 13, in which the spring member-blocking means (24) forms part of a securing element (22).

15. Injection syringe (1) according to one or more of the preceding claims 10-14, in which the spring member (23) is in a prestressed state.

## Patentansprüche

1. Injektionsspritze (1) mit zurückziehbarer Nadel (2), die zumindest aufweist:
- einen Flüssigkeitsbehälter (3) mit einer Auslassöffnung (4),
- eine Nadel (2) mit einer Nadelhalterung (8), die an der Auslassöffnung (4) des Flüssigkeitsbehälters (3) befestigt ist,
- einen Kolben (5), der im Flüssigkeitsbehälter (3) bewegbar ist und einen Kolbenkopf (6) aufweist, an dem eine Kolbenstange (7) befestigt ist,
wobei die Nadelhalterung (8) der Nadel (2) und der Kolbenkopf (6) Verbindungsmittel aufweisen, die so ausgebildet sind, dass sie durch Bewegen des Kolbens (5) zur Auslassöffnung (4) hin ausfallsicher miteinander verbindbar sind,
**dadurch gekennzeichnet, dass**
eine Blockiereinrichtung (14) vorgesehen ist, die so ausgelegt ist, dass die Nadelhalterung (8) in der Auslassöffnung (4) blockiert wird, wobei die Blockiereinrichtung (14) in Form eines oder mehrerer elastischer Ansätze an der Nadelhalterung (8) ausgebildet ist, die in entsprechenden Ausnehmungen (18) im Flüssigkeitsbehälter (3) aufgenommen sind,
wobei die Blockiereinrichtung (14) nur durch Zurückziehen der Nadelhalterung (8) und der Nadel (2) in den Flüssigkeitsbehälter (3) durch Wegbewegen des Kolbens (5) von der Auslassöffnung (4) entblockt werden kann, nachdem die Nadelhalterung (8) mit dem Kolbenkopf (6) verbunden wurde, und wobei die Verbindungsmittel der Nadelhalterung (8) der Nadel (2) mindestens zwei Rippen (15) umfassen, die an einem Verbindungspunkt (16) auf der zum Kolbenkopf (6) hin liegenden Seite miteinander verbunden sind, wobei der eine Ansatz oder die mehreren Ansätze an den Rippen (15) vorgesehen sind,
und die Rippen (15) der Nadelhalterung (8) näher zusammen bewegbar sind, wenn die Nadel (2) durch Wegbewegen des Kolbens (5) von der Auslassöffnung (4) in den Flüssigkeitsbehälter (3) zurückgezogen wird, nachdem der Kolbenkopf (6) mit der Nadelhalterung (8) verbunden wurde.

2. Injektionsspritze (1) nach Anspruch 1, bei der die Rippen (15) federnd sind.

3. Injektionsspritze (1) nach einem der vorhergehenden Ansprüche, bei der die Rippen (15) eine durchgehende Öffnung (21) teilweise umgeben.

4. Injektionsspritze (1) nach einem der vorhergehenden Ansprüche, bei welcher der Verbindungspunkt (16) eine gelenkige Verbindung ist, insbesondere ein integriertes Gelenk.

5. Injektionsspritze (1) nach einem oder mehreren der vorhergehenden Ansprüche, bei der mindestens eine der Rippen (15) der Nadelhalterung (8) eine Krümmung in Richtung der Längsachse des Flüssigkeitsbehälters (3) aufweist.

6. Injektionsspritze (1) nach einem oder mehreren der vorhergehenden Ansprüche, bei der mindestens eine der Rippen (15) der Nadelhalterung (8) ein Verbindungselement (17) aufweist, das zur Wand des Flüssigkeitsbehälters (3) hin gerichtet ist.

7. Injektionsspritze (1) nach Anspruch (6), bei der mindestens zwei der Rippen (15) der Nadelhalterung (8) ein Verbindungselement (17) aufweisen, das zur Wand des Flüssigkeitsbehälters (3) hin gerichtet ist, wobei sich mindestens eines der Verbindungselemente (17) im Vergleich zu dem mindestens einen anderen Verbindungselement (17) in einem unterschiedlichen Abstand vom Verbindungspunkt (16) befindet.

8. Injektionsspritze (1) nach einem oder mehreren der vorhergehenden Ansprüche, bei der die Verbindungsmittel der Nadelhalterung (8) drei Rippen (15) umfassen, die am Verbindungspunkt (16) auf der zum Kolbenkopf (6) hin liegenden Seite miteinander verbunden sind.

9. Injektionsspritze (1) nach einem oder mehreren der vorhergehenden Ansprüche, bei der die Nadelhalterung (8) auf ihrer zum Kolbenkopf (6) hin liegenden Seite mit einem Verbindungselement (20) zur Verbindung mit dem Kolbenkopf (6) versehen ist, das vorzugsweise am Verbindungspunkt (16) angebracht ist.

10. Injektionsspritze (1) nach einem oder mehreren der vorhergehenden Ansprüche, die ferner ein federndes Element aufweist, das die Nadelhalterung (8) mit der Nadel (2) zwangsweise in den Flüssigkeitsbehälter (3) bewegt, nachdem die Blockiereinrichtung (14) entblockt wurde.

11. Injektionsspritze (1) nach Anspruch 10, bei der das federnde Element (23) einen Teil der Nadelhalterung (8) bildet.

12. Injektionsspritze (1) nach Anspruch 10 oder 11, bei der das federnde Element (23) durch eine Blockiereinrichtung (24) für das federnde Element blockiert ist.

13. Injektionsspritze (1) nach Anspruch 12, bei der die Blockiereinrichtung (24) für das federnde Element zum Zweck der Blockierung des federnden Elements (23) mit einer Schutzkappe (25) zusammenwirkt.

14. Injektionsspritze (1) nach Anspruch 12 oder 13, bei der die Blockiereinrichtung (24) für das federnde Element einen Teil eines Sicherungselements (22) bildet.

15. Injektionsspritze (1) nach einem oder mehreren der vorhergehenden Ansprüche 10-14, bei der sich das federnde Element (23) in einem vorgespannten Zustand befindet.

## Revendications

1. Seringue d'injection (1) avec une aiguille rétractable (2), comprenant au moins :
- un conteneur de liquide (3) comportant une ouverture de sortie (4) ;
- une aiguille (2) avec un support d'aiguille (8) qui est fixé sur l'ouverture de sortie (4) du conteneur de liquide (3) ;
- un piston (5) mobile à l'intérieur dudit conteneur de liquide (3) et comportant une tête de piston (6), à laquelle une tige de piston (7) est fixée, dans laquelle le support d'aiguille (8) de l'aiguille (2) et la tête de piston (6) comprennent des moyens d'accouplement qui sont conçus de sorte qu'ils puissent être couplés sans possibilité d'erreur l'un à l'autre en déplaçant le piston (5) vers l'ouverture de sortie (4) ;
**caractérisée en ce que**
des moyens de blocage (14) sont prévus, lesquels sont conçus pour bloquer le support d'aiguille (8) dans l'ouverture de sortie (4), les moyens de blocage (14) étant conçus sous la forme d'une ou de plusieurs pattes élastiques sur le support d'aiguille (8) qui sont reçues dans des évidements (18) correspondants dans le conteneur de liquide (3),
lesquels moyens de blocage (14) ne peuvent être débloqués que par une rétraction du support d'aiguille (8) et de l'aiguille (2) dans le conteneur de liquide (3) par un mouvement du piston (5) à l'opposé de l'ouverture de sortie (4) après l'accouplement du support d'aiguille (8) et de la tête de piston (6),
et dans laquelle les moyens d'accouplement du support d'aiguille (8) de l'aiguille (2) comprennent au moins deux nervures (15) qui sont reliées l'une à l'autre en un point de liaison (16) du côté qui fait face à la tête de piston (6), lesdites une ou plusieurs pattes étant prévues sur les nervures (15),
lesquelles nervures (15) du support d'aiguille (8) peuvent être rapprochées les unes des autres, lorsque l'aiguille (2) est rétractée dans le conteneur de liquide (3) par un mouvement du piston (5) à l'opposé de l'ouverture de sortie (4) après l'accouplement de la tête de piston (6) et du support d'aiguille (8).

2. Seringue d'injection (1) selon la revendication 1, dans laquelle les nervures (15) sont résilient.

3. Seringue d'injection (1) selon l'une des revendications précédentes, dans laquelle les nervures (15) entourent partiellement une ouverture continue (21).

4. Seringue d'injection (1) selon l'une des revendications précédentes, dans laquelle le point de liaison (16) comprend une liaison articulée, en particulier une articulation intégrale.

5. Seringue d'injection (1) selon une ou plusieurs des revendications précédentes, dans laquelle au moins l'une des nervures (15) du support d'aiguille (8) présente une courbure dans la direction de l'axe longitudinal du conteneur de liquide (3).

6. Seringue d'injection (1) selon une ou plusieurs des revendications précédentes, dans laquelle au moins l'une des nervures (15) du support d'aiguille (8) comprend un élément d'accouplement (17) qui est dirigé vers la paroi du conteneur de liquide (3).

7. Seringue d'injection (1) selon la revendication 6, dans laquelle au moins deux des nervures (15) du support d'aiguille (8) comprennent un élément d'accouplement (17) qui est dirigé vers la paroi du conteneur de liquide (8), au moins l'un des éléments d'accouplement (17) étant à une distance différente du point de liaison (16) comparé audit au moins un autre élément d'accouplement (17).

8. Seringue d'injection (1) selon une ou plusieurs des revendications précédentes, dans laquelle les moyens d'accouplement du support d'aiguille (8) comprennent trois nervures (15) qui sont reliées les unes aux autres au niveau du point de liaison (16) du côté qui fait face à la tête de piston (6).

9. Seringue d'injection (1) selon une ou plusieurs des revendications précédentes, dans laquelle le support d'aiguille (8) est pourvu, de son côté qui fait face à la tête de piston (6), d'un élément d'accouplement (20) pour un accouplement à la tête de piston (6), lequel élément d'accouplement (20) est de préférence relié au point de liaison (16).

10. Seringue d'injection (1) selon une ou plusieurs des revendications précédentes, dans laquelle la seringue d'injection (1) comprend également un élément formant ressort (23) pour forcer le support d'aiguille (8) avec l'aiguille (2) dans le conteneur de liquide (3) après le déblocage des moyens de blocage (14).

11. Seringue d'injection (1) selon la revendication 10, dans laquelle l'élément formant ressort (23) fait partie du support d'aiguille (8).

12. Seringue d'injection (1) selon la revendication 10 ou 11, dans laquelle l'élément formant ressort (23) est bloqué par des moyens de blocage d'élément formant ressort (24).

13. Seringue d'injection (1) selon la revendication 12, dans laquelle les moyens de blocage d'élément formant ressort (24) interagissent avec un couvercle de protection (25) en vue de bloquer l'élément formant ressort (23).

14. Seringue d'injection (1) selon la revendication 12 ou 13, dans laquelle les moyens de blocage d'élément formant ressort (24) font partie d'un élément de fixation (22).

15. Seringue d'injection (1) selon une ou plusieurs des revendications précédentes 10 à 14, dans laquelle l'élément formant ressort (23) est dans un état précontraint.
